# EUROPÄISCHE PATENTSCHRIFT

(11) **EP 2 767 295 B1**
(45) Veröffentlichungstag und Bekanntmachung des Hinweises auf die Patenterteilung: **04.11.2015**
(21) Anmeldenummer: 13193269.1
(22) Anmeldetag: 18.11.2013
(51) Int. Cl.: A61L 31/02, A61L 31/10, A61L 31/14

(54) **Biokorrodierbares Implantat mit korrosionshemmender Beschichtung**
Biocorrodible implant with anti-corrosion coating
Implant biocorrodable doté d'un revêtement anticorrosion

(30) Priorität: 13.02.2013 US 201361763955 P
(43) Veröffentlichungstag der Anmeldung: 20.08.2014
(73) Patentinhaber: Biotronik AG, 8180 Bülach (CH)
(72) Erfinder: Wittchow, Eric, 91710 Gunzenhausen (DE)
(74) Vertreter: Randoll, Sören

(56) Entgegenhaltungen:
- US-A1- 2006 271 168
- US-A1- 2009 240 323

## Beschreibung

Die Erfindung betrifft ein biokorrodierbares Implantat in Form eines Stents auf Basis von Magnesium oder einer biokorrodierbaren Magnesiumlegierung, dessen Oberfläche eine korrosionshemmende Beschichtung aufweist.

Insbesondere die Implantation von Stents hat sich als eine der wirkungsvollsten therapeutischen Maßnahmen bei der Behandlung von Gefäßerkrankungen etabliert. Stents haben den Zweck, in Hohlorganen eines Patienten eine Stützfunktion zu übernehmen. Stents herkömmlicher Bauart weisen dazu eine filigrane Tragstruktur aus metallischen Streben auf, die zur Einbringung in den Körper zunächst in einer komprimierten Form vorliegt und am Ort der Applikation aufgeweitet wird. Einer der Hauptanwendungsbereiche solcher Stents ist das dauerhafte oder temporäre Weiten und Offenhalten von Gefäßverengungen, insbesondere von Verengungen (Stenosen) der Herzkranzgefäße. Daneben sind beispielsweise auch Aneurysmenstents bekannt, die vorrangig zur Abdichtung des Aneuryrismas dienen.

Stents besitzen eine Umfangswandung von ausreichender Tragkraft, um das verengte Gefäß im gewünschten Maße offen zu halten, und einen rohrförmigen Grundkörper durch den der Blutfluss ungehindert weiterläuft. Die Umfangswandung wird in der Regel von einer gitterartigen Tragstruktur gebildet, die es erlaubt, den Stent in einem komprimierten Zustand mit kleinem Außendurchmesser bis zur zu behandelnden Engstelle des jeweiligen Gefäßes einzuführen und dort beispielsweise mit Hilfe eines Ballonkatheters soweit aufzuweiten, dass das Gefäß den gewünschten, vergrößerten Innendurchmesser aufweist. Alternativ besitzen Form-Gedächtnis-Materialien wie Nitinol die Fähigkeit zur Selbstexpansion, wenn eine Rückstellkraft wegfällt, die das Implantat auf einem kleinen Durchmesser hält. Die Rückstellkraft wird in der Regel durch einen Schutzschlauch auf das Material ausgeübt.

Der Stent besitzt einen Grundkörper aus einem Implantatwerkstoff. Ein Implantatwerkstoff ist ein nicht lebendes Material, das für eine Anwendung in der Medizin eingesetzt wird und mit biologischen Systemen in Wechselwirkung tritt. Grundvoraussetzungen für den Einsatz eines Werkstoffes als Implantatwerkstoff, der bei bestimmungsgemäßen Zweck mit der Körperumgebung in Kontakt steht, ist dessen Körperverträglichkeit (Biokompatibilität). Unter Biokompatibilität wird die Fähigkeit eines Werkstoffes verstanden, in einer spezifischen Anwendung eine angemessene Gewebereaktion hervorzurufen. Dies beinhaltet eine Anpassung der chemischen, physikalischen, biologischen und morphologischen Oberflächeneigenschaften eines Implantates an das Empfängergewebe mit dem Ziel einer klinisch erwünschten Wechselwirkung. Die Biokompatibilität des Implantatswerkstoffs ist weiterhin abhängig vom zeitlichen Ablauf der Reaktion des Biosystems in das implantiert wird. So treten relativ kurzfristig Reizungen und Entzündungen auf, die zu Gewebeveränderungen führen können. Biologische Systeme reagieren demnach in Abhängigkeit von den Eigenschaften des Implantatswerkstoffs in verschiedener Weise. Entsprechend der Reaktion des Biosystems können die Implantatswerkstoffe in bioaktive, bioinerte und degradierbare/resorbierbare Werkstoffe unterteilt werden. Implantatwerkstoffe umfassen Polymere, metallische Werkstoffe und keramische Materialien (zum Beispiel als Beschichtung). Biokompatible Metalle und Metalllegierungen für Permanentimplantate beinhalten beispielsweise rostfreie Stähle (zum Beispiel 316L), Kobaltbasislegierungen (zum Beispiel CoCrMo-Gußlegierungen, CoCrMo-Schmiedelegierungen, CoCrWNi-Schmiedelegierungen und CoCrNiMo-Schmiedelegierungen), Reintitan und Titanlegierungen (zum Beispiel cp Titan, TiAl6V4 oder TiAl6Nb7) und Goldlegierungen. Im Bereich biokorrodierbarer Implantate, insbesondere Stents, wird der Einsatz von Magnesium oder Reineisen sowie biokorrodierbaren Basislegierungen der Elemente Magnesium, Eisen, Zink, Molybdän und Wolfram vorgeschlagen.

Für die Zwecke der vorliegenden Erfindung sind lediglich metallische Implantatwerkstoffe von Interesse, die ganz oder in Teilen aus Magnesium oder biokorrodierbaren Magnesiumlegierungen bestehen.

Ein Problem des Einsatzes biokorrodierbarer Magnesiumlegierungen ist die rasche Degradation des Materials in physiologischer Umgebung. Sowohl die Grundlagen der Magnesiumkorrosion als auch eine große Anzahl von technischen Verfahren zur Verbesserung des Korrosionsverhaltens (im Sinne einer Verstärkung des Korrosionsschutzes) sind aus dem Stand der Technik bekannt. Bekannt ist beispielsweise, dass der Zusatz von Yttrium und/oder weiteren Seltenerdmetallen einer Magnesiumlegierungen einen leicht erhöhten Korrosionswiderstand in Meerwasser verleiht.

Ein Ansatzpunkt zur Verbesserung des Korrosionsverhaltens sieht vor, eine korrosionsschützende Schicht auf dem aus Magnesium oder einer Magnesiumlegierung bestehenden Formkörper zu erzeugen. Bekannte Verfahren zur Erzeugung einer korrosionsschützenden Schicht wurden bisher aus dem Gesichtspunkt eines technischen Einsatzes des Formkörpers - jedoch nicht medizintechnischen Einsatzes in biokorrodierbaren Implantaten in physiologischer Umgebung - entwickelt und optimiert. Diese bekannten Verfahren umfassen: das Aufbringen von Polymeren oder anorganischen Deckschichten, das Erzeugen einer Emaille, die chemische Konversion der Oberfläche, Heißgasoxidation, Anodisierung, Plasmaspritzen, Laserstrahl-Umschmelzen, PVD-Verfahren, Ionenimplantation oder Lackieren.

Herkömmliche technische Einsatzgebiete von Formkörpern aus Magnesiumlegierungen außerhalb der Medizintechnik erfordern in der Regel eine weitgehende Unterbindung korrosiver Prozesse. Dementsprechend ist die Zielstellung der meisten technischen Verfahren eine vollständige Eliminierung korrosiver Prozesse. Dagegen sollte die Zielstellung zur Verbesserung des Korrosionsverhaltens von biokorrodierbaren Magnesiumlegierungen nicht in der vollständigen Unterbindung, sondern nur in der Hemmung korrosiver Prozesse liegen. Schon aus diesem Grunde eignen sich die meisten bekannten Verfahren zur Erzeugung von Korrosionsschutzschichten auf Magnesium nicht. Ferner müssen für einen medizintechnischen Einsatz auch toxikologische Aspekte berücksichtigt werden. Des Weiteren sind korrosive Prozesse stark von dem Medium abhängig, in dem sie ablaufen, und daher dürfte eine Übertragbarkeit der unter herkömmlichen Umweltbedingungen auf technischem Gebiet gewonnenen Erkenntnisse zum Korrosionsschutz auf die Prozesse in physiologischer Umgebung nicht uneingeschränkt möglich sein. Schließlich dürfen bei einer Vielzahl von medizinischen Implantaten auch die der Korrosion zugrunde liegenden Mechanismen von üblichen technischen Anwendungen des Werkstoffs abweichen. So werden beispielsweise Stents, chirurgisches Material oder Clips im Gebrauch mechanisch verformt, sodass der Teilprozess der Spannungsrisskorrosion beim Abbau dieser Formkörper erhebliche Bedeutung haben dürfte.

Der Grundkörper von Stents wird im Gebrauch lokal unterschiedlich starken plastischen Verformungen unterworfen. Konventionelle Verfahren zur Hemmung der Korrosion, wie beispielsweise die Generierung einer dichten Magnesiumoxid-Deckschicht, sind hier nicht zielführend. Die keramischen Eigenschaften einer solchen Deckschicht würden zu einem lokalen Abplatzen der Deckschicht führen. Die Korrosion fände damit in unkontrollierter Weise statt und es bestünde insbesondere die Gefahr, dass in den mechanisch besonders beanspruchten Bereichen des Implantats die Korrosion forciert ist.

Der Auftrag nicht degradierbarer, polymerer Passivierungsschichten kann zwar den Abbau des Implantats hemmen, jedoch konterkariert er die Grundidee eines völlig degradierbaren Implantats, denn im Körper verbleibt das polymere Material der Passivierungsschicht. Eine vielversprechende Alternative ist daher die Verwendung von biodegradierbaren Polymeren als Werkstoff für eine Passivierungsschicht für Implantate auf Basis von biodegradierbaren Magnesiumwerkstoffen.

In US 2006/0271168 ist ein beschichteter biodegradierbarer Stent beschrieben. Aus US 2009-0240323 A1, US 2010-0076544 A1 und US 2011/0076319 A1 ist es bekannt, einen Magnesium-Stent mit einer biodegradierbaren polymeren Beschichtung als Passivierungsschicht zu beschichten. Hierdurch soll der Abbau des Grundkörpers aus Magnesium beziehungsweise einer Magnesiumlegierung verlangsamt werden. Als biodegradierbare polymere Beschichtungsmaterialien kommen beispielsweise Poly(lactide), Polyhydroxyalkanoate (insbesondere Polyhydroxyvalerat), Polycaprolactone, aliphatische Polyester, aromatische Copolyester und Polyesteramide in Frage. Trotz dieser vielversprechenden Ansätze, ist es bisher nicht zufriedenstellend gelungen, die Degradation des metallischen Grundkörpers des Stents hinreichend zu verzögern und dabei gleichzeitig allen weiteren Anforderungen an die Beschichtung, wie insbesondere eine hinreichende Bruchdehnung, zu genügen.

Eines oder mehrere der geschilderten Probleme des Standes der Technik lassen mit Hilfe des erfindungsgemäßen Implantats beheben oder zumindest zu mindern. Der Stent besitzt einen Grundkörper aus Magnesium oder einer biokorrodierbaren Magnesiumlegierung und eine den Grundkörper bedeckende korrosionshemmende Passivierungsschicht. Die Passivierungsschicht zeichnet sich dadurch aus, dass sie ein 3-Polyhydroxyalkanoat enthält und das 3-Polyhydroxyalkanoat ein Homomer oder Copolymer mit einem Polymersegment der Formel (1) ist: 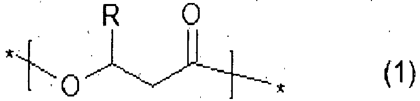 mit R = Propyl, Butyl, Pentyl oder Hexyl. Vorzugsweise ist das 3-Polyhydroxyalkanoat ausgewählt aus der Gruppe umfassend Polyhydroxyhexanoat (PHHx), Polyhydroxyheptanoat (PHHp), Polyhydroxyoctonoat (PHO) und Polyhydroxynonanoat (PHN).

Polyhydroxyalkanoate (PHA) sind natürlich vorkommende wasserunlösliche und lineare Polyester. Die Polymere sind biologisch abbaubar und werden zur Herstellung von Kunststoffen verwendet, aber auch als biodegradierbarer Werkstoff für Medizinprodukte, wie Nahtmaterialien, für Implantate und für pharmazeutische Depotpräparate. Die Biosynthese von PHA liefert je nach verwendetem Mikroorganismus und Kultivierungsbedingungen verschiedene Homo- oder Copolyester, die sich in ihren Eigenschaften unterscheiden. Die Polymere sind in der Regel UV-stabil, vertragen Verarbeitungstemperaturen von bis zu 180 °C und zeigen eine geringe Permeation von Wasser. Die einfachste und am häufigsten vorkommende Form der PHA ist das fermentativ hergestellte Poly(R-3-hydroxybutyrat) (Polyhydroxybuttersäure, PHB oder Poly(3HB). PHB und weitere kurzkettige PHA sind allerdings relativ spröde und steif.

Der Erfindung liegt die Erkenntnis zugrunde, dass der Einsatz langkettiger Polyhydroxyalkanoate mit von C6 bis C9 abgeleiteten Polyhydroxyfettsäuren als Beschichtungsmaterialien für Implantate, (insbesondere für Stents), aus Magnesium oder Magnesiumlegierungen besondere Vorteile mit sich bringt. So ist zum einen die Wasserundurchlässigkeit der Passivierungsschicht sehr gering, was zu einer temporären Hemmung der Korrosion der darunter liegenden metallischen Grundkörpers führt. Zum anderen zeigt das Material ein für die Zwecke besonders vorteilhaftes Verhalten, insbesondere ist die Bruchdehnung des polymeren Materials hinreichend hoch. Bei der Expansion von Stents kommt es daher beispielsweise nicht zu einer Mikrorissbildung in der Passivierungsschicht, was ein inhomogenes Abbauverhalten des Implantates bedingen könnte.

Die Passivierungsschicht kann weitere Hilfsstoffe und vorzugsweise auch Wirkstoff enthalten, die nach der Implantation freigesetzt werden.

### Ausführungsbeispiel - Beschichtung eines absorbierbaren Metallstents aus einer Magnesiumlegierung mit einem wirkstoffbeladenem Polyhydroxyhexanoat (PHHx)

Der Stent wird von Staub und Rückständen gereinigt und in eine geeignete Stentbeschichtungsapparatur (DES Coater, Eigenentwicklung Fa. Biotronik) eingespannt. Für die Beschichtung wird eine Lösung von 0,1 Gew.% PHHx und 0,05 Gew.% eines pharmakologischen Wirkstoffes (zum Beispiel Rapamycin) in Chloroform angesetzt. Mit Hilfe eines Airbrush Systems wird der sich drehende Stent unter konstanten Umgebungsbedingungen (Raumtemperatur; 42% Luftfeuchte) halbseitig beschichtet. Bei einem Düsenabstand von 20mm ist ein 18mm langer Stent nach ca. 10min beschichtet. Nach Erreichen der vorgegebenen Schichtmasse wird der Stent 5 min bei Raumtemperatur getrocknet, ehe nach Drehen des Stents und erneutem Einspannen die unbeschichtete Seite auf dieselbe Weise beschichtet wird. Der fertig beschichtete Stent wird für 24h bei 80°C in einem Vakuumofen getrocknet.

## Patentansprüche

1. Implantat mit einem Grundkörper aus Magnesium oder einer biokorrodierbaren Magnesiumlegierung und einer den Grundkörper bedeckenden korrosionshemmenden Passivierungsschicht, wobei die Passivierungsschicht ein 3-Polyhydroxyalkanoat enthält und das 3-Polyhydroxyalkanoat ein Homomer oder Copolymer mit einem Polymersegment der Formel (1) ist: 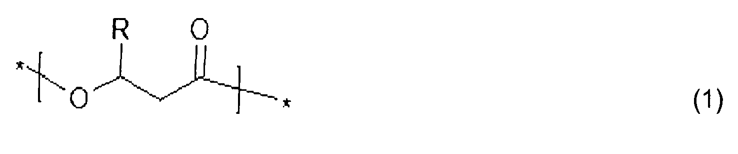 mit R = Propyl, Butyl, Pentyl oder Hexyl, wobei das Implantat ein Stent ist.

2. Implantat nach Anspruch 1, bei dem das 3-Polyhydroxyalkanoat ausgewählt ist aus der Gruppe umfassend Polyhydroxyhexanoat (PHHx), Polyhydroxyheptanoat (PHHp), Polyhydroxyoctonoat (PHO) und Polyhydroxynonanoat (PHN).

## Claims

1. An implant having a main body formed from magnesium or a biocorrodible magnesium alloy and a corrosion-inhibiting passivation layer covering the main body, wherein the passivation layer contains a 3-polyhydroxyalkanoate and the 3-polyhydroxyalkanoate is a homomer or copolymer with a polymer segment of formula (1): 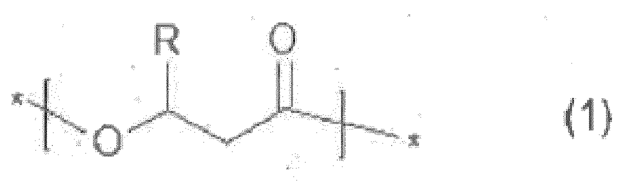 with R = propyl, butyl, pentyl or hexyl, wherein the implant is a stent.

2. The implant according to claim 1, in which the 3-polyhydroxyalkanoate is selected from the group comprising polyhydroxyhexanoate (PHHx), polyhydroxyheptanoate (PHHp), polyhydroxyoctanoate (PHO) and polyhydroxynonanoate (PHN).

## Revendications

1. Implant avec un corps de base en magnésium ou en un alliage de magnésium biocorradable et une couche de passivation empêchant la corrosion recouvrant le corps de base, où la couche de passivation contient un 3-polyhydroxy alcanoate et le 3-polyalcanoate est un homomère ou un copolymère avec un segment de polymère selon la formule (I) : 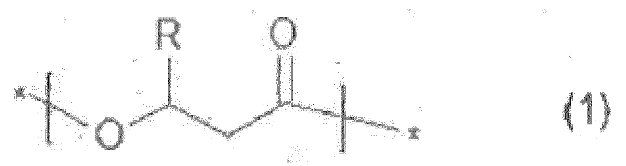 avec R = un groupe propyle, butyle, pentyle ou hexyle, où l'implant est un stent.

2. Implant selon la revendication 1, chez lequel le 3-polyhydoxy alcanoate est choisi dans le groupe comprenant un polyhydroxy hexanoate (PHHx)), un polyhydroxy heptanoate (PHHp), un polyhydroxy octonoate (PHO) et un polyhydroxy nonanoate (PHN).
